# FASCICULE DE BREVET EUROPEEN

(11) **EP 3 769 676 B1**
(45) Date de publication et mention de la délivrance du brevet: **16.02.2022**
(21) Numéro de dépôt: 20186692.8
(22) Date de dépôt: 20.07.2020
(51) Int. Cl.: A61B 5/11, A61B 5/00

(54) **SYSTEME ET PROCEDE DE DETECTION DE PRESENCE DE PERSONNES ALITEES**
SYSTEM UND VERFAHREN ZUR PRÄSENZERKENNUNG VON BETTLÄGERIGEN PERSONEN
SYSTEM AND METHOD FOR DETECTING THE PRESENCE OF BEDRIDDEN PERSONS

(30) Priorité: 23.07.2019 FR 1908355
(43) Date de publication de la demande: 27.01.2021
(73) Titulaire: WINNCARE FRANCE, 85670 Saint-Paul-Mont-Penit (FR)
(72) Inventeur: JOUET, Pascal, 85670 SAINT-PAUL-MONT-PENIT (FR); BARTHELAT, Julien, 85670 SAINT-PAUL-MONT-PENIT (FR); HEBRARD, Jocelyn, 85670 SAINT-PAUL-MONT-PENIT (FR); GEAY, Pierre, 85670 SAINT-PAUL-MONT-PENIT (FR)
(74) Mandataire: Demulsant, Xavier

(56) Documents cités:
- EP-A1- 2 659 832
- EP-A1- 3 415 134
- US-A- 5 144 284

## Description

L'invention a trait au domaine des systèmes de suivi de l'activité d'une personne occupant un lit, et en particulier de la détection de présence d'une personne dans un lit.

Dans toute la suite de la description, par "activité", on désigne tant la position de la personne (allongée, ou assise) que la présence de la personne dans le lit.

L'invention trouve une application dans les lits, en particulier les lits médicalisés installés en établissement hospitalier, institutions médicalisées, maison de retraite, maison de repos ou domicile.

L'invention est notamment à destination d'individus alités, par exemple des patients atteints de pathologies physiques et/ou mentales, des personnes physiquement diminuées. L'invention vise en particulier les sujets âgés.

Les personnes âgées sont particulièrement sujettes aux chutes. Selon une expertise de l'Institut national de la santé et de la recherche médicale (Activité physique et prévention des chutes chez les personnes âgées. Expertise collective, Inserm, 2015*)* chaque année, environ 30% des plus de soixante-cinq ans et 50% des plus de quatre-vingt ans chutent. En 2013, plus de 9000 décès de personnes âgées de plus de soixante-cinq ans sont imputables à une chute, le taux de mortalité augmentant avec l'âge (Thélot et al., Surveillance épidémiologique des chutes chez les personnes âgées, 2013*).*

En institution, il a été constaté que les chutes sont plus fréquentes en établissement d'hébergement des personnes âgées dépendantes (EHPAD) qu'à domicile. L'incidence moyenne est évaluée à 1,7 chute par lit et par an (de 0,6 à 3,6) pour les résidents d'EHPAD, alors qu'elle n'est que de 0,65 (de 0,3 à 1,6) pour les personnes âgées vivant à domicile (Rubenstein, Falls in older people: epidemiology, risk factors and strategies for prevention, Age and Ageing, 37, 2006*).*

En EHPAD, il a été observé que les personnes chutent le plus souvent lors des transferts d'une chaise ou du lit à une position debout ou inversement (Fall Prevention un Nursing Home, Prévention des chutes en EHPAD, La Revue de Gériatrie, Tome 40, N°6, juin 2015*).*

De manière à prévenir la survenue de chute lors de ces transferts, il a été proposé la contention des individus, par exemple les gilets et sangles thoraciques, ou les barrières de lit.

Cependant, les répercussions physiques, psychologiques et sociales sont importantes (voir par exemple l'évaluation des pratiques professionnelles dans les établissements de santé, Agence Nationale d'Accréditation et d'Evaluation en Santé (ANAES), Octobre 2000, P. 13*).* Par ailleurs, l'efficacité de telles mesures n'a pas été prouvée (Capezuti et al., The relationship between physical restraint removal and falls and injuries among nursing home residents, Journal of Gerontology, 1998*).* L'utilisation de la contention physique est donc limitée à des cas bien particuliers, et dans des conditions fortement encadrées, afin de limiter son impact sur le sujet alité. En outre, l'utilisation de telles méthodes peut engendrer de l'anxiété ainsi qu'un sentiment de culpabilité chez le soignant ou autre personne aidante (dans le cas par exemple d'un maintien à domicile de la personne âgée).

Aussi, les pratiques actuelles tendent à traiter prioritairement les causes des chutes, en œuvrant notamment à améliorer l'état physique de l'individu alité par une prise en charge personnalisée et adaptée.

La détection des chutes est tout particulièrement recherchée, non seulement pour permettre de détecter et de suivre les sujets "chuteurs", mais également pour intervenir le plus rapidement possible en cas de chute.

Néanmoins, la mise en œuvre d'une telle démarche préventive est complexe, principalement en raison du manque de personnel dans les EHPAD (23 postes de soignants pour 100 places en EHPAD selon une étude menée par la Direction de Recherche, des Etudes de l'Evaluation et des Statistiques publiée en juin 2018 dans Etudes & Résultats n°1067, https://drees.solidarites-sante.gouv.fr/IMG/pdf/er1067.pdf), qui plus est déjà très accaparé par les tâches de soins quotidiens. La difficulté est accrue en période de nuit ou week-end, les soignants étant dans de telles périodes souvent en effectif réduit.

De plus, les exigences élevées de certaines familles par rapport aux soignants en termes de suivi qualitatif de l'activité de l'individu alité, en particulier du suivi des chutes, ne peuvent dans certains cas pas être satisfaites. Une telle situation peut dans certains cas engendrer des contentieux, les familles des patients pouvant être amenées à faire engager la responsabilité des EHPAD dans le cas d'une chute entrainant des conséquences sur leur proche.

Une solution pour permettre le suivi de la personne dans un lit, ainsi que des chutes, tout en mobilisant un personnel réduit consiste en l'installation d'un système de vidéosurveillance dans la chambre de l'individu alité, le système étant pourvu d'un moyen d'analyse pour détecter certains types de mouvements. Néanmoins, un tel système présente l'inconvénient d'être onéreux, et d'être lié à la pièce où le patient se trouve. Ainsi, en cas de changement de chambre, l'activité de l'individu alité n'est plus suivie. En outre, compte-tenu des législations en vigueur liées au respect de la vie privée, et à la dignité de l'individu alité, l'utilisation de vidéos collectées est fortement réglementée, limitant les possibilités d'exploitation des images collectées, ainsi que leur durée de conservation dans le temps.

Le suivi de l'activité des personnes âgées hospitalisées à domicile est également une problématique importante pour la personne accompagnant le sujet alité. Une telle personne aidante n'a pas forcément l'expérience, ni le temps pour suivre l'activité de la personne alitée, et détecter tout événement anormal, par exemple un temps de lever anormalement long.

Le document FR3062207 (Fogale Nanotech) divulgue une couche compressible comprenant une électrode de mesure et une électrode de garde, une telle couche étant associée à un module d'analyse qui peut permettre la mesure de la position du corps de la personne sur la couche, un suivi du déplacement ou la détection de chute. Néanmoins, les électrodes sont directement intégrées à la couche, ce qui empêche une fabrication simple, et un entretien aisé. Une telle couche ne permet par ailleurs pas l'utilisation de matelas de prévention d'apparition des escarres, par exemple un matelas gaufré en mousse.

Le document EP 2659832 A1 (Univisio Oy) divulgue un capteur capacitif permettant la détection des mouvements d'un individu sur un matelas, le capteur comprenant une pluralité d'électrodes disposées longitudinalement dans le lit. Cependant, un tel capteur ne permet pas d'avoir une indication précise de la position, ni même des parties du corps de l'individu qui se déplacent.

Le document EP 3415134 A1 (Winncare) décrit un dispositif pour la prévention de formation d'escarres, et permettant la détection d'absence de l'individu alité, le dispositif comprenant des capteurs capacitifs disposés entre le support et l'individu, les capteurs étant disposés sur une housse. Cependant, rien n'est prévu pour faciliter le lavage de la housse, ni pour rendre les capteurs imperceptibles pour l'individu allongé sur le matelas muni d'une telle housse.

L'invention se propose de résoudre les inconvénients de l'art antérieur.

Un premier objet de l'invention est de proposer un système de suivi de l'activité d'un patient dans un lit, permettant d'alerter une tierce personne sur l'absence d'activité, le changement de position et l'absence d'un patient.

Un deuxième objet de l'invention est de proposer un tel système de suivi qui soit fiable.

Un troisième objet de l'invention est de proposer un tel système de suivi comprenant un drap-housse offrant un ressenti de confort similaire à celui d'un drap-housse conventionnel.

Un quatrième objet de l'invention est de proposer un tel système de détection comprenant un drap-housse lavable, de la même manière qu'un drap-housse conventionnel.

Un cinquième objet de l'invention est de proposer un tel système de détection comprenant un drap-housse permettant l'utilisation de moyens de prévention des escarres.

Un sixième objet de l'invention est de proposer un lit comprenant un tel système de détection.

Un septième objet de l'invention est de proposer une méthode de suivi de l'activité d'une personne alitée à l'aide du système de suivi tel que présenté ci-dessus.

A cet effet, il est proposé, en premier lieu, un système selon la revendication 1

Un tel système permet un suivi efficace d'une personne alitée, et délivre une alerte pour le cas où la personne alitée se lève. Un tel système offre l'avantage de n'avoir d'impact défavorable ni sur le confort de la personne, ni sur les moyens de prévention (par exemple les dispositifs de prévention des escarres) et de soin apportés à l'individu occupant le lit.

Diverses caractéristiques supplémentaires peuvent être prévues pour le système, seules ou en combinaison, selon les revendications dépendantes.

Il est proposé, en deuxième lieu, un lit comprenant le système de suivi tel que présenté ci-dessus.

Il est proposé, en troisième lieu, une méthode de suivi de l'activité d'une personne alitée sur un lit, suivant la revendication 7.

D'autres caractéristiques et avantages de l'invention apparaîtront plus clairement et de manière concrète à la lecture de la description ci-après de modes de réalisation, laquelle est faite en référence aux dessins annexés dans lesquels :
- la figure 1 représente une vue schématique en perspective d'un lit muni d'un système de suivi de l'activité d'une personne dans un lit,
- la figure 2 représente une vue schématique d'un tel système de suivi,
- la figure 3 représente une vue schématique d'un drap équipant un tel système de suivi,
- la figure 4 représente un graphique représentant des exemples de longueurs actives exprimées en mm, mesurées pour chaque capteur pour un individu présent dans le lit, et un individu absent du lit,
- la figure 5 représente un graphique représentant des exemples de longueurs actives exprimées en mm, mesurées pour chaque capteur pour un individu en position allongée et un individu en position assise sur le lit,

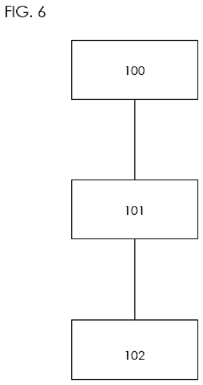

- la figure 6 est un ordinogramme illustrant les étapes relatives à un mode de réalisation d'un système de suivi de l'activité d'une personne dans un lit.

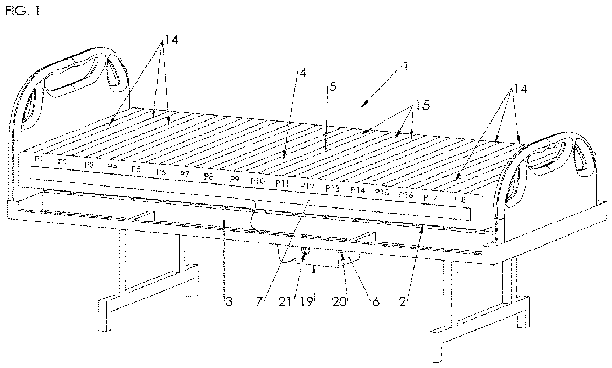

En se référant à la figure 1, il est représenté un lit 1 muni d'un matelas 2, le lit 1 comprenant un système 3 de suivi de l'activité chez un individu alité dans un tel lit 1. Dans toute la suite de la description, l'on désigne le système 3 de suivi de l'activité d'une personne dans un lit par "système 3 de suivi".

Le système 3 de suivi comprend un drap 4, muni de capteurs 5, et une unité 6 de traitement, qui sont reliés l'un à l'autre, par exemple par l'intermédiaire d'un faisceau 7.

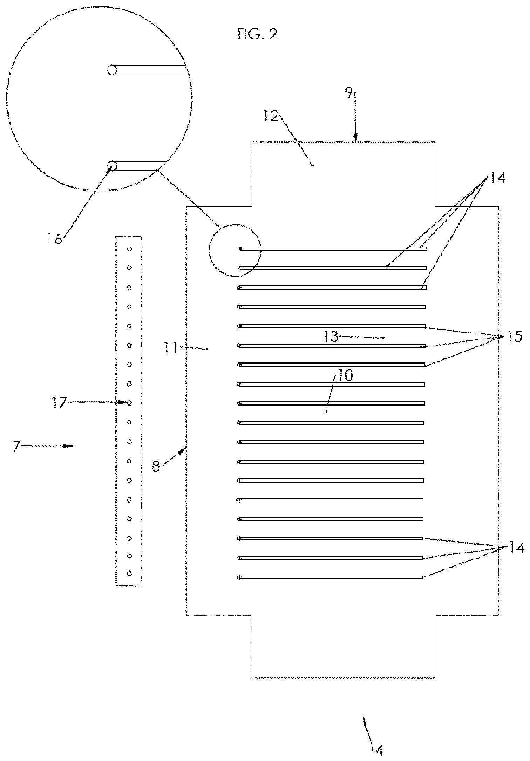

L'on se rapporte à la figure 2 représentant plus en détail le système 3 de suivi.

Le drap 4 présente une silhouette sensiblement rectangulaire, comprenant un côté 8 longitudinal et un côté 9 transversal, le côté 8 longitudinal étant de longueur supérieure au côté 9 transversal.

Le drap 4 comprend une face 10 de contact comprenant les capteurs 5 et une ou plusieurs faces 11 de fixation. La face 10 de contact est destinée à être positionnée sur une face supérieure du matelas 2 du lit 1, tandis que les faces 11 de fixation s'étendent entre le bord du lit 1 et le matelas.

La face 10 de contact est destinée à être disposée entre l'individu alité et la face supérieure du matelas 2. Ainsi, si l'individu est sur le lit 1, la face 10 de contact est recouverte partiellement ou complètement par le corps de l'individu.

Dans des modes de réalisation, un moyen élastique est placé en partie inférieure du drap 4, formant ainsi un drap housse dont le bonnet est adapté au matelas.

Dans des modes de réalisation, une housse de protection supplémentaire (non représentée sur les figures) recouvre le drap 4 du système 3 de suivi, la personne alitée n'entrant pas directement en contact avec le drap 4 du système 3 de suivi. La housse de protection comporte avantageusement une fermeture à glissière sur trois côtés, l'ouverture de la housse permettant l'accès aux capteurs pour le montage du faisceau 7.

Une telle housse de protection, avantageusement réalisée en matériau imperméable, évite les souillures du drap 4, liée par exemple aux liquides corporels de l'individu alité (transpiration ou urine).

Une telle housse de protection permet également d'éviter un contact direct entre le drap 4 et le sujet alité, ce qui préserve la sensation de confort de l'individu alité. L'utilisation d'une telle housse de protection permet d'éviter le risque d'allergie que pourrait occasionner un contact prolongé entre les capteurs 5 et la peau de l'individu alité, dans le cas par exemple où les capteurs 5 comprennent de l'argent à leur surface.

Dans le mode de réalisation représenté, les faces 11 de fixation sont agencées sur des rabats 12 rectangulaires s'étendant sur chaque côté de la face 10 de contact. Le drap 4 ainsi mis en place se positionne de manière à ce que la face 10 de contact couvre bien le matelas 2. Une telle mise en place est réalisable par toute personne, y compris par une personne ignorant l'existence de capteurs 5 au sein du drap 4. L'utilisation du drap 4 est donc facilitée.

Dans d'autres modes de réalisation, non représentés, le drap 4 est un drap-housse comprenant une unique face 11 de fixation entourant la face 10 de contact et munie d'un lien élastique. Un tel drap-housse est facilement mis en place, et reste maintenu en position sur le matelas 2. Par ailleurs, un tel drap-housse ne peut pas se déplacer si le sujet alité bouge.

Dans d'autres modes de réalisation, une sur-housse est formée par l'assemblage en un seul élément d'un drap 4 tel que représenté en figure 2 et d'une housse de protection. La housse de protection comporte avantageusement une fermeture à glissière, l'ouverture de la sur-housse permettant l'accès aux capteurs pour le montage du faisceau 7.

Comme présenté ci-dessus, le drap 4 comprend une pluralité de capteurs 5, disposés sur la face 10 de contact, destinés à être recouverts par le corps de l'individu alité lorsque dans le lit 1.

Conformément à l'invention, les capteurs 5 s'étendent également dans une ou plusieurs faces 11 de fixation, au moins partiellement, permettant de connecter le drap 4 avec le restant du système 3 de suivi, sans que l'individu alité ne soit gêné.

Les capteurs 5 sont ménagés avantageusement parallèlement entre eux, par exemple par l'intermédiaire de bandes 13, 14 rectilignes souples, qui s'étendent avantageusement de manière parallèles. Dans le mode de réalisation représenté, les bandes 13, 14 couvrent l'intégralité de la face 10 de contact.

Les capteurs 5, qui conformément à l'invention se matérialisent sous forme de bandes 13, 14 sont avantageusement disposés dans la largeur du drap 4, c'est-à-dire dans une direction sensiblement parallèles au côté 9 transversal. Ainsi, lorsqu'un individu est allongé sur le lit 1, les bandes 13, 14 sont disposées en regard de parties de corps. Des bandes 13 extrêmes positionnées à l'extrémité de la face 10 de contact sont situées en regard de la tête et des pieds de l'individu alité, tandis que les bandes 14 centrales disposées au centre de la face 10 de contact sont en regard et/ou à proximité de l'abdomen de l'individu alité lorsque alité.

Les capteurs 5, sous forme de bandes 13, 14, sont avantageusement disposés équidistants les uns des autres, permettant d'éviter d'avoir des parties du drap 4 vides, c'est-à-dire ne permettant pas de détection. De cette façon, le drap 4 est utilisable pour toute morphologie d'individu.

Dans d'autres modes de réalisation non représentés, les bandes 13, 14 ne présentent entre elles un écartement variable. De cette manière, une plus grande précision de captation est obtenue, permettant par exemple de réaliser une détection plus fiable de la position de la personne alitée.

Les capteurs 5 sont des capteurs 5 capacitifs, chacun des capteurs 5 définissant une première armature. Les capteurs 5 forment ensemble une surface 15 captante. Une deuxième armature est constituée du corps de l'individu alité recouvrant la surface 15 captante lorsque assis ou allongé sur le lit 1. Une capacité est ainsi créée entre les armatures.

Dans le mode de réalisation représenté, les capteurs 5 relèvent chacun une capacité, l'ensemble des valeurs de capacités relevées par les capteurs sont proportionnelles à des longueurs de bandes 13, 14 actives, c'est-à-dire la portion de bande recouverte par le corps.

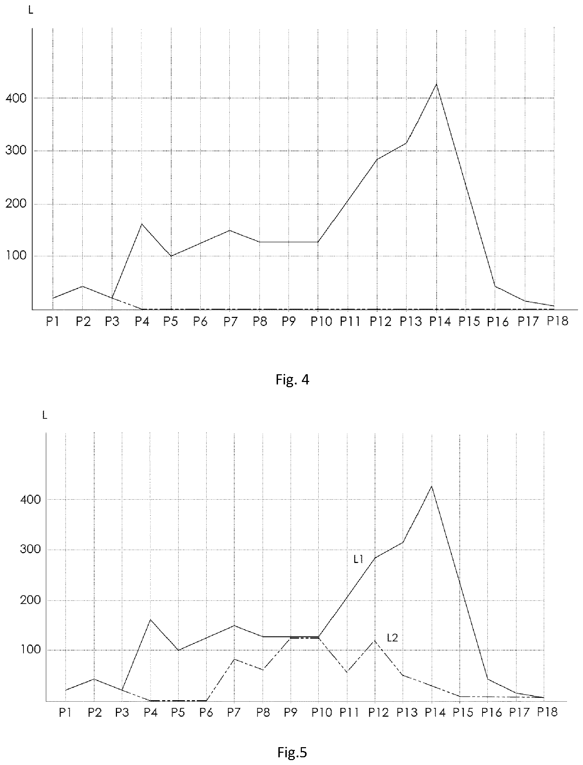

L'on se réfère aux figures 4 et 5 représentant les longueurs actives mesurées par chacun des capteurs 5. Sans que cela soit limitatif, dix-huit capteurs 5 sont présents dans le drap, permettant la réalisation de dix-huit points de mesure P1 à P18, le point de mesure P1 étant à proximité de la tête de lit, tandis que le point de mesure P18 est à proximité du pied de lit.

Dans le mode de réalisation représenté, le drap 4 comprend dix-huit bandes 13, 14 présentant une largeur de 90 mm, espacées entre elles de 100 mm. Dans d'autres modes de réalisation, non représentés, le drap 4 comprend douze bandes 13, 14 présentant une largeur de 90 mm, et espacées les unes des autres de 145 mm. Un compromis est ainsi atteint entre le nombre de bandes 13, 14 et la qualité de précision de la position de l'individu alité.

Les capteurs 5 sont solidarisés au drap 4. Les capteurs sont avantageusement des bandes 13, 14 conductrices, réalisées par exemple en tissus conducteur.

Les capteurs 5 sont par exemple collés, imprimés ou cousus sur le drap 4.

Dans le mode de réalisation représenté, les capteurs 5 sont tissés au sein du drap 4. De cette façon, le drap 4 présente une texture semblable à un drap conventionnel c'est-à-dire sans capteur. Un sujet alité ne perçoit donc pas la présence des capteurs 5. La résistance à l'usure des capteurs 5 est accrue par rapport à des capteurs imprimés, permettant une bonne tenue dans le temps, et une bonne résistance aux souillures.

Avantageusement, le drap 4 est réalisé dans un matériau suffisamment résistant pour pouvoir être nettoyé à haute température.

Avantageusement, les capteurs 5 comprennent des fils réalisés à partir d'un matériau conducteur d'électricité. De cette façon, un capteur réalisé à partir de fil peut être facilement intégré dans un drap 4, et n'a aucun impact négatif tant sur le confort de l'individu alité, que sur la possibilité d'utilisation de moyen de soins préventifs et curatifs, par exemple pour la prise en charge des plaies de pression.

Dans un exemple de mise en œuvre, un capteur 5 comprend un fil polyamide enduit d'argent. Un tissu composé de telle fibre est facile à refendre pour couper en bandes et à coudre, ce qui facilite l'obtention du drap 4. Un textile composé de tel fils offre une résistance accrue au lavage, ce qui n'impacte pas le traitement du linge dans l'institution ou l'établissement de soin. Enfin, un tissu réalisé avec de tels fils demeure souple, et n'est pas rugueux, ce qui est avantageux en terme de ressenti de confort pour l'individu alité, en évitant par exemple tout relief.

Le drap 4 comprend par ailleurs des premiers points 16 de raccordement disposés à une extrémité de chaque capteur 5, afin de permettre le raccordement des capteurs 5 à l'unité 6 de traitement par l'intermédiaire du faisceau 7 sur des deuxièmes points 17 de raccordement complémentaires. Ainsi, lorsqu'un premier point 16 de raccordement est mâle, le deuxième point 17 de raccordement est femelle.

Dans le mode de réalisation représenté, les premiers points 16 de raccordement sont tous mâles et les deuxièmes points 17 de raccordement sont tous femelles, ce qui permet de faciliter la fabrication, et permet la réversibilité de connexion du faisceau 7.

Dans d'autres modes de réalisation non représentés, des premiers points 16 de raccordement sont mâles, les autres premiers points 16 de raccordement sont femelles. De manière complémentaire, des deuxièmes points 17 de raccordement sont femelles, et d'autres deuxièmes points 17 de raccordement sont mâles.

Les premiers points 16 de raccordement et les deuxièmes sont des éléments métalliques s'engageant l'un dans l'autre par pression, par exemple des boutons pressions. De tels boutons offrent l'avantage d'être peu encombrant, permettant le positionnement des premiers points 16 de raccordement sur une face 11 de fixation. De tels boutons permettent un accrochage ou un décrochage facile, rapide et robuste du faisceau 7 sur le drap 4. L'entretien du drap 4 est ainsi facilité.

Le faisceau 7 a pour objet de relier les bandes 13, 14 à l'unité 6 de traitement.

Le faisceau 7 englobe avantageusement un bandeau souple muni de deuxièmes points 17 de raccordement, qui sont connectés à l'unité 6 de traitement. La mise en place et le retrait du faisceau 7 est facile d'exécution. Le faisceau 7 est peu encombrant et ne gêne par conséquent pas le sujet alité. Le faisceau 7 peut être également recouvert par un drap supplémentaire, et est ainsi invisible pour l'individu alité.

Dans le mode de réalisation représenté, l'unité 6 de traitement est fixée sur le lit 1, avantageusement sous le lit 1.

L'unité 6 de traitement comprend les composants électroniques aptes à collecter, analyser, et si besoin transmettre les données mesurées par les capteurs 5.

L'unité 6 de traitement se présente avantageusement sous la forme d'un boitier 19, permettant de protéger les composants électroniques.

L'unité 6 de traitement comprend des moyens 20 de fixation magnétiques, tels que des aimants permanents disposés sur le boitier 19, permettant une fixation sur le cadre métallique du lit 1 réalisable rapidement, sans outil, et en ne requérant qu'un apprentissage limité. Ainsi, le système 3 de suivi est amovible, ce qui peut être avantageux si le sujet alité est amené à changer de lit 1.

Avantageusement, l'unité 6 de traitement est fixée sous le lit 1, comme il peut être observé sur la figure 1. De cette manière, l'unité 6 de traitement n'est pas visible et accessible à l'individu alité.

De cette manière, l'unité 6 de traitement n'est pas à portée de main de l'individu alité. Le sujet alité n'a donc pas la possibilité de manipuler l'unité 6 de traitement, ce qui est favorable notamment pour les personnes souffrant de pathologies psychiatriques. Le risque de dégradation du boitier suite à une manipulation inadéquate est éliminé.

Avantageusement, le boitier 19 présente une teinte identique ou similaire à la couleur de la structure métallique du lit. L'unité 6 de traitement est ainsi discrète, ce qui évite d'attirer l'attention de l'individu alité sur le boitier 19.

Dans un mode de réalisation avantageux, afin de pallier le déficit de prises de courant dans les institutions, l'unité 6 de traitement comprend une prise électrique 21 femelle, disposée sur le boitier 19.

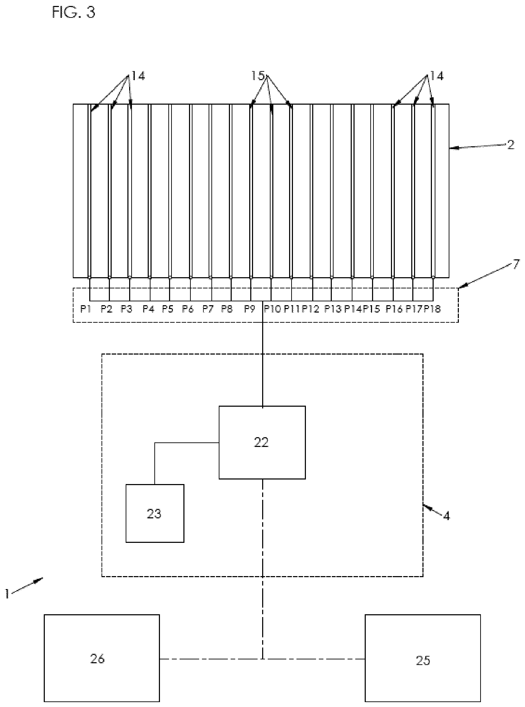

Comme il peut être observé sur la figure 3, l'unité 6 de traitement comprend par exemple un calculateur 22, un support mémoire 23, et un émetteur. Le calculateur permet notamment la collecte des données transmises par les capteurs 5, et l'analyse de telles données. L'émetteur permet notamment la transmission de messages d'alerte via par exemple une interface vers un terminal 25, 26.

Dans les modes de réalisation représentés, l'émetteur utilise un protocole de transmission sans fil, tel que le bluetooth, le wifi ou le réseau téléphonique mobile selon la norme 2G ou supérieure.

Dans d'autres modes de réalisation, non représentés, l'unité 6 de traitement est connecté par voie filaire au terminal 25, 26, par exemple par l'intermédiaire d'un câble réseau.

Dans le mode de réalisation représenté, un terminal est un terminal 25 fixe par exemple un ordinateur.

Selon d'autres mises en œuvre, le terminal est un terminal 26 mobile tel qu'une tablette ou un smartphone. Un terminal 26 mobile offre l'avantage de permettre une intervention rapide du personnel soignant ou d'une tierce personne aidante.

Dans des mises en œuvre préférentielles, l'unité 6 de traitement est connectée à plusieurs terminaux 25, 26, qui peuvent être tous fixes, tous mobiles, ou à la fois mobiles et fixes.

L'unité 6 de traitement transmet des informations relatives à l'état du système 3 de suivi, ainsi que des messages d'alarmes correspondants à des situations prédéfinies qui nécessitent par exemple une intervention rapide de l'équipe soignante ou d'une tierce personne aidante. Ainsi des messages d'alarmes peuvent être envoyés en relation avec des situations prédéterminées.

Selon un mode de réalisation, pour une situation d'une personne alitée non autonome, un message d'alarme de non-présence de l'individu, est envoyé si l'individu ne repose pas sur le lit pendant un temps prédéterminé.

Selon un mode de réalisation, pour une situation d'une personne alitée autonome, un message d'alarme de non-présence de l'individu est envoyé si l'individu est absent du lit après une durée prédéterminée. Une telle durée prédéterminée est définie comme représentant un temps anormal de sortie de lit, et nécessitant un contrôle par le personnel soignant ou d'une tierce personne aidante afin de vérifier si l'individu alité n'a pas été victime d'une chute.

Dans le cas par exemple d'une utilisation dans un établissement, un logiciel, par exemple une interface de visualisation installée sur un serveur dédié, permet la réception des messages, et la gestion du ou des systèmes 3 de détection installés. La gestion d'un parc de plusieurs lits 1, chacun munis d'un système 3 de suivi est ainsi facilitée pour le personnel soignant d'un établissement.

De manière à permettre une lecture efficace, l'interface permet par exemple un affichage des informations relatives à chaque système 3 de suivi, par exemple par utilisation d'un système de couleurs.

Avantageusement, l'interface permet un suivi des personnes alitées sur une période prédéterminée. Il est ainsi possible de déterminer à quel moment le sujet s'est levé, mis en position assise ou le temps d'immobilité de l'individu alité. Le personnel d'un établissement peut ainsi individualiser de manière optimale la prise en charge pour l'individu alité.

Un historique des données collectées pour chaque personne est ainsi possible, permettant d'obtenir un suivi de l'activité de l'individu alité sur le moyen-terme voire le long-terme. Un tel outil permet le comptage des épisodes d'alertes, permettant au personnel aidant de se focaliser sur les individus alités à risque de chute. Il est également possible pour les utilisateurs de détecter les personnes se levant fréquemment, par exemple la nuit, de tels sujets étant considérés comme ayant un risque de chute accru.

Dans un mode de réalisation préférentiel, l'unité 6 de traitement, soit directement, soit par exemple par l'intermédiaire de l'interface, envoie des messages d'alarmes sur des terminaux 25, 26 mobiles détenus par exemple par le personnel soignant. Ainsi, même lorsque le soignant n'a pas accès à l'interface, par exemple en cas d'intervention auprès d'un individu, il peut être par exemple informé de la présence d'une sortie de lit chez un autre individu. De cette manière, le temps d'intervention pour prendre en charge l'individu alité est ainsi réduit.

Avantageusement, il est possible à l'aide du terminal 26 mobile d'avoir accès à l'interface, ce qui permet d'avoir une vue globale du parc. Ainsi, même si l'effectif du personnel est limité (par exemple la nuit), une personne peut intervenir rapidement sans devoir revenir vers le terminal 25 fixe. La personne intervenante optimise ainsi ses déplacements dans l'établissement, ce qui lui permet de passer plus de temps avec les personnes alitées, et gagner du temps.

L'on décrit à présent un exemple de fonctionnement du système 3 de suivi d'activité et en particulier pour le changement de position d'un individu dans un lit.

Dans une étape 100 de relevé, les capteurs 5 procèdent à une mesure de longueur L active de capteurs 5. Un tel relevé s'effectue de manière périodique, la période de mesure étant réglable.

Dans une étape 101 de comparaison, les longueurs L actives relevées sont comparées avec des longueurs de référence.

Pour détecter le changement de position dans le lit 1, des longueurs L actives d'une mesure sont comparées avec des valeurs de longueur de capteur de référence, stockées par exemple dans l'unité 6 de traitement.

Selon un exemple de réalisation, les valeurs de référence des capteurs 5 sont nulles ou une valeur seuil de longueur active L est fixée, par exemple à 50, ce qui correspond à une absence d'une personne dans le lit, comme il peut être observé figure 4 (courbe en trait mixte).

Selon un exemple de réalisation, les valeurs de références des capteurs 5 correspondent à la présence d'une personne dans le lit en position allongée. Un exemple de telles longueurs est donné par la courbe L1 de la figure 5, ou à la courbe en trait plein de la figure 4. Les valeurs mesurées en continu par douze capteurs (P4 à P15) dépassent une valeur seuil de longueur active, par exemple égale à 50, correspondant à la présence d'une personne dans le lit en position allongée.

Selon un mode de réalisation, les valeurs de référence des capteurs 5 de pieds ou de tête sont sensiblement nulles, permettant de modéliser une situation de personne assise. Un exemple de telles longueurs est donné par la courbe L2 de la figure 5. Les valeurs mesurées par six capteurs (P7 à P12) dépassent une valeur seuil de longueur active, par exemple égale à 50, correspondant à l'occupation de la longueur du lit par une personne assise.

Lorsqu'un nombre prédéfini de capteurs 5 présentent une longueur L active supérieure à une des longueurs de seuil prédéterminée, un message d'alerte est envoyé à un terminal 25, 26 au cours d'une étape 102 de déclenchement.

Avantageusement, le message d'alerte précise le type d'alerte, en fonction de la situation détectée.

Avantageusement, durant une telle étape 102 de déclenchement, l'unité 6 de traitement identifie le système 3 de suivi qui détecte une alerte, avec un identifiant de lit. De cette manière, le message est envoyé aux terminaux accompagné de l'identifiant du lit, ce qui permet au personnel de savoir instantanément le lit en situation, et par extension la personne susceptible d'avoir chuté.

Avantageusement, l'unité 6 de traitement envoie un message au terminal 25, 26 de sorte à permettre l'acquittement du message d'alerte.

Le drap 4 peut avantageusement être placé directement sur le matelas, et le drap 4 peut être protégé par une housse étanche. Le drap 4 n'est ainsi pas en contact direct avec la personne alitée.

Le système 3 de suivi offre de nombreux avantages, en particulier:
- une durée de vie maximisée, grâce aux capteurs 5 tissés sur le drap 4,
- les capteurs 5 ne sont pas perceptibles par la personne allongée sur le drap 4, la sensation de confort de l'individu alité est préservée,
- le système 3 de suivi n'empêche pas d'utiliser des moyens de prise en charge des escarres, par exemple des matelas spécifiques,
- une sureté d'utilisation, l'unité 6 de traitement étant mise à distance de l'individu alité,
- une absence d'impact sur l'entretien, le drap 4 pouvant être nettoyé de la même manière que la literie non munie de capteurs 5,
- une fabrication économique du drap 4, du fait de l'utilisation de trames filaire pour la réalisation des capteurs.

## Revendications

1. Système (3) de suivi de l'activité d'une personne alitée comprenant:
- un drap (4) apte à recouvrir un matelas (2) d'un lit (1), le drap (4) présentant une silhouette sensiblement rectangulaire, comprenant un côté (8) longitudinal et un côté (9) transversal, le côté (8) longitudinal étant de longueur supérieure au côté (9) transversal, le drap (4) comprenant une face (10) de contact comprenant des capteurs (5), la face (10) de contact étant destinée à être positionnée sur une face supérieure du matelas (2), entre la personne alitée et la face supérieure du matelas (2),
- des capteurs (5) capacitifs disposés sur le drap (4) dans une direction sensiblement parallèle au côté (9) transversal, les capteurs (5) étant apte à mesurer une longueur (L) active correspondant à une longueur de capteur (5) couverte par la personne alitée, le drap (4) comprenant une pluralité de capteurs (5) disposés sur la face (10) de contact, les capteurs (5) se présentant sous la forme de bandes (13, 14) parallèles, une unité (6) de traitement configurée pour collecter et analyser les longueurs (L) de capteurs actives, et les comparer avec des longueurs de référence,
- l'unité (6) de traitement et les capteurs (5) étant reliés par l'intermédiaire d'un faisceau (7), des premiers points (16) de raccordement étant disposés à une extrémité de chaque capteur (5) afin de permettre le raccordement des capteurs (5) à l'unité (6) de traitement par l'intermédiaire de deuxièmes points (17) de raccordement complémentaires d'un faisceau (7), les capteurs (5) étant solidarisés au drap (4), le drap (4) comprenant au moins une face (11) de fixation destinée à s'étendre entre le bord du lit et le matelas, **caractérisé en ce que** les capteurs (5) s'étendent dans au moins une face (11) de fixation, les premiers points (16) et les deuxièmes points (17) de raccordement étant des éléments métalliques s'engageant l'un dans l'autre par pression, les premiers points (16) de raccordement étant positionnés sur une face (11) de fixation du drap (4).

2. Système (3) de suivi selon la revendication précédente, **caractérisé en ce qu'**un capteur (5) est solidarisé au drap (4) par tissage, impression ou collage.

3. Système (3) de suivi selon l'une quelconque des revendications précédentes, **caractérisé en ce que** l'unité (6) de traitement comprend un boitier (19) muni d'un émetteur (24) apte à autoriser la communication entre le système (3) de suivi et un terminal (25, 26), par utilisation d'un protocole de communication filaire ou sans fil.

4. Système (3) de suivi selon l'une quelconque des revendications précédentes, **caractérisé en ce que** l'unité (6) de traitement comprend une prise électrique (21) femelle, et des moyens (20) de fixation magnétiques.

5. Système (3) de suivi selon l'une quelconque des revendications précédentes, **caractérisé en ce que** le drap (4) est un drap-housse.

6. Lit (1) comprenant le système (3) de suivi selon l'une quelconque des revendications précédentes.

7. Méthode de suivi de l'activité d'une personne alitée sur un lit selon la revendication 6, l'unité (6) de traitement mettant en œuvre:
- une étape (100) de relevé de longueurs actives (L) de capteurs,
- une étape (101) de comparaison des longueurs actives (L) avec des longueurs de référence prédéterminées,
- une étape (102) de déclenchement d'une alerte si un nombre prédéterminé de longueurs actives (L) sont supérieures à des longueurs de référence prédéterminées.

## Patentansprüche

1. System (3) zur Verfolgung der Aktivität einer bettlägerigen Person, umfassend:
- ein Laken (4), das geeignet ist, um eine Matratze (2) eines Bettes (1) abzudecken, wobei das Laken (4) eine deutlich rechteckige Silhouette aufweist, umfassend eine längliche Seite (8) und eine querverlaufende Seite (9), wobei die längliche Seite (8) eine größere Länge aufweist als die querverlaufende Seite (9), wobei das Laken (4) eine Kontaktseite (10) umfasst, umfassend Sensoren (5) wobei die Kontaktseite (10) dazu bestimmt ist, auf einer oberen Seite der Matratze (2) zwischen der bettlägerigen Person und der oberen Seite des Matratze (2) angeordnet zu sein,
- kapazitive Sensoren (5), die auf dem Laken (4) in einer zur querverlaufenden Seite (9) deutlich parallelen Richtung angeordnet sind, wobei die Sensoren (5) geeignet sind, um eine aktive Länge (L) zu messen, die einer von der bettlägerigen Person abgedeckten Sensorlänge (5) entspricht, wobei das Laken (4) eine Vielzahl von Sensoren (5) umfasst, die auf der Kontaktseite (10) angeordnet sind, wobei die Sensoren (5) die Form von parallelen Bändern (13, 14), eine Verarbeitungseinheit (6), die zum Erfassen und Analysieren der aktiven Sensorlänge (L) und zu deren Vergleich mit Bezugslängen ausgestaltet ist, aufweisen,
- wobei die Verarbeitungseinheit (6) und die Sensoren (5) mittels eines Bündels (7) miteinander verbunden sind, wobei erste Anschlusspunkte (16) an einem Ende jedes Sensors (5) angeordnet sind, um den Anschluss der Sensoren (5) an die Verarbeitungseinheit (6) mittels zweiter, zu einem Bündel (7) komplementärer Anschlusspunkte (17) zu ermöglichen,
wobei die Sensoren (5) fest mit dem Laken (4) verbunden sind, umfassend wenigstens eine Befestigungsseite (11), die dazu bestimmt ist, sich zwischen dem Rand des Bettes und der Matratze zu erstrecken,
**dadurch gekennzeichnet, dass** die Sensoren (5) sich auf wenigstens auf einer Befestigungsseite (11) erstrecken, wobei die ersten Anschlusspunkte (16) und die zweiten Anschlusspunkte (17) Metallelemente sind, die unter Druck ineinander eingreifen, wobei die ersten Anschlusspunkte (16) auf einer Befestigungsseite (11) des Lakens (4) angeordnet sind.

2. System (3) zur Verfolgung gemäß dem voranstehenden Anspruch, **dadurch gekennzeichnet, dass** ein Sensor (5) per Verweben, Aufdruck oder Verkleben fest mit dem Laken (4) verbunden ist.

3. System (3) zur Verfolgung gemäß irgendeinem der voranstehenden Ansprüche, **dadurch gekennzeichnet, dass** die Verarbeitungseinheit (6) ein Gehäuse (19) umfasst, das mit einem Sender (24) versehen ist, der geeignet ist, um die Kommunikation zwischen dem System (3) zur Verfolgung und einem Terminal (25, 26) durch die Verwendung eines Kommunikationsprotokolls mit oder ohne Draht zu erlauben.

4. System (3) zur Verfolgung gemäß irgendeinem der voranstehenden Ansprüche, **dadurch gekennzeichnet, dass** die Verarbeitungseinheit (6) eine elektrische Buchse (21) und magnetische Befestigungsmittel (20) umfasst.

5. System (3) zur Verfolgung gemäß irgendeinem der voranstehenden Ansprüche, **dadurch gekennzeichnet, dass** das Laken (4) ein Spannbettlaken ist.

6. Bett (1), umfassend das System (3) zur Verfolgung gemäß irgendeinem der voranstehenden Ansprüche.

7. Verfahren zur Verfolgung der Aktivität einer bettlägerigen Person auf einem Bett gemäß Anspruch 6, wobei die Verarbeitungseinheit (6) umsetzt:
- einen Schritt (100) zum Erfassen von aktiven Sensorlängen (L),
- einen Schritt (101) zum Vergleichen der aktiven Längen (L) mit vorbestimmten Bezugslängen,
- einen Schritt (102) zum Auslösen eines Alarms, wenn eine vorbestimmte Anzahl von aktiven Längen (L) größer ist als vorbestimmte Bezugslängen.

## Claims

1. A system (3) for tracking the activity of a bedridden person comprising:
- a sheet (4) capable of covering a mattress (2) of a bed (1), the sheet (4) having a substantially rectangular outline, comprising a longitudinal side (8) and a transverse side (9), the longitudinal side (8) being of a greater length than the transverse side (9), the sheet (4) comprising a contact face (10) comprising sensors (5), the contact face (10) being to be positioned on an upper face of the mattress (2), between the bedridden person and the upper face of the mattress (2),
- capacitive sensors (5) disposed on the sheet (4) in a direction substantially parallel to the transverse side (9), the sensors (5) being capable of measuring an active length (L) corresponding to a length of sensor (5) covered by the bedridden person, the sheet (4) comprising a plurality of sensors (5) disposed on the contact face (10), the sensors (5) being in the form of parallel strips (13, 14), a processing unit (6) configured to collect and analyse the active lengths (L) of the sensors, and compare them with reference lengths,
- the processing unit (6) and the sensors (5) being connected through à beam (7), first connection points (16) being disposed at an end of each sensor (5) in order to enable connection of the sensors (5) to the processing unit (6) through second complementary connection points (17) of a beam (7),
the sensors (5) being secured to the sheet (4), the sheet (4) comprising at least one fastening face (11) for extending between the edge of the bed and the mattress,
**characterised in that** the sensors (5) extend into at least one fastening face (11), the first (16) and second (17) connection points being metal elements engaging into one another by pressure, the first connection points (16) being positioned on a fastening face (11) of the sheet (4).

2. The tracking system (3) according to the preceding claim, **characterised in that** a sensor (5) is secured to the sheet (4) by weaving, printing or bonding.

3. The tracking system (3) according to any of the preceding claims, **characterised in that** the processing unit (6) comprises a casing (19) provided with a transmitter (24) capable of authorising communication between the tracking system (3) and a terminal (25, 26), by using a wired or wireless communication protocol.

4. The tracking system (3) according to any of the preceding claims, **characterised in that** the processing unit (6) comprises a power socket (21), and magnetic fastening means (20).

5. The tracking system (3) according to any of the preceding claims, **characterised in that** the sheet (4) is a fitted sheet.

6. A bed (1) comprising the tracking system (3) according to any of the preceding claims.

7. A method for tracking the activity of a bedridden person on a bed according to claim 6, the processing unit (6) implementing:
- a step (100) of reading out active lengths (L) of sensors,
- a step (101) of comparing the active lengths (L) with predetermined reference lengths,
- a step (102) of triggering an alert if a predetermined number of active lengths (L) are greater than predetermined reference lengths.
